# EUROPEAN PATENT APPLICATION

(11) **EP 2 500 334 A1**
(43) Date of publication of application: **19.09.2012**
(21) Application number: 10827818.5
(22) Date of filing: 25.06.2010
(51) Int. Cl.: C07C 1/20, C07C 11/02, C07C 11/04, C07C 11/06

(54) **PROCESS FOR PRODUCING LOWER ALKENES WITH METHANOL OR DIMETHYL ETHER**

(30) Priority: 04.11.2009 CN 200910210635
(71) Applicant: Sure Billion Corporation Limited, Beijing 100082 (CN)
(72) Inventor: WEI, Xiaobo, Beijing 100082 (CN); LIU, Weiwei, Hong Kong (CN); MEI, Ling, Hong Kong (CN)
(74) Representative: Held, Stephan
(86) International application number: PCT/CN2010/074453
(87) International publication number: WO 2011/054204

(57) **Abstract**

Disclosed is a process for producing light olefins from methanol or dimethyl ether. In the process, the fresh catalyst or the regenerated catalyst is pretreated to deposit a certain amount of coke onto its interior pore surface in advance, to reduce the generation of alkane and heavy olefins, so as to increase the selectivities to ethylene and propylene; and during the production of the light olefins, the pretreated catalyst is used for catalyzing the methanol or the dimethyl ether to produce light olefins. The process can achieve higher yields of ethylene and propylene.

## Description

### Technical Field

This invention relates to a process for producing light olefins, particularly to a process for producing light olefins from methanol or dimethyl ether. The process is useful for the preparation of chemical material.

### Background Art

Light olefins, such as ethylene and propylene, serve as important feed stock for the production of chemicals. Light olefins are currently produced mainly by the cracking of light oil, such as naphtha and light diesel oil, etc. However, with the petroleum gradually becomes short of supplies, the process for producing ethylene and propylene from abundant raw material such as coal and natural gas get attention increasingly over the world.

In 1976, Mobile Company developed a methanol to gasoline (MTG) process using ZSM-5 mesoporous molecular sieve as a catalyst, primarily to synthesize gasoline from methanol. It has been found at the same time that the catalyst can be used for converting methanol to light olefins directly. In 1980's, UCC Company successfully developed SAPO molecular sieves, in which SAPO-34 molecular sieve catalyst exhibits an excellent catalytic performance, a very high selectivity to light olefins, and a very high activity in the methanol to olefin (MTO) process, however this catalyst become deactivated as the deposition of coke onto the surface of inner pores of the catalyst over a period of time.

CN116478A discloses a method for preparing ethylene, propylene and other low olefins from methanol or dimethyl ether, wherein the catalyst was used and then regenerated in a dense-phase circulating fluidized bed reactor. The catalyst is regenerated by burning out the deposited coke on its surface to resume its activity, thereby being circulated in the reactor and regenerator, and producing low olefins such as ethylene and propylene continuously.

There is a significant induction period for SAPO-34 molecular sieve catalyst during its use. In the induction period, the selectivity is low to olefin and high to alkane. With the elapse of the reaction time, the selectivity to lower olefin increases gradually, then the selectivity and activity keep at a high level after the induction period, and then the activity of the catalyst decreases rapidly with the further elongation of time.

US7045672B2 and US7057083B2 disclose processes for pretreating catalyst with dimethyl ether and C4-C7 olefins respectively, wherein the dimethyl ether and C4-C7 olefins are derived from the subsequent separation and refining of MTO process. After the catalyst is pretreated with dimethyl ether or C4-C7 olefins, a co-catalyst containing hydrocarbon is formed within the catalyst, achieving a higher yield of ethylene and propylene. It is noted in the inventions that the pretreated catalyst has a coke content of no greater than 2 wt %, more preferably no greater than 1.5 wt %, more preferably no greater than 1 wt %, and most preferably no greater than 0.5 wt %, based on the weight of the molecular sieve.

With the development of researches on catalysts and processes for producing light olefins from methanol or dimethyl ether, it is highly desirable to increase the catalyst's selectivity to ethylene and propylene in the processes for producing light olefins from methanol or dimethyl ether, in order to achieve a yield of ethylene and propylene as high as possible.

### Summary of the Invention

The object of this invention is a process for producing light olefins from methanol or dimethyl ether (Chia-Tai Pretreatment Relay Process, abbreviated as CPR process). A pretreatment step is added ahead of the reactor, where catalyst is pretreated to deposit a certain amount of coke onto its interior pore surface in advance, to reduce the generation of alkane and higher olefins, at the same time to increase the selectivity to ethylene and propylene. The use of the pretreated catalyst in the reactor insures that the catalysts operates under optimal condition, and thus achieve higher yields of ethylene and propylene.

In order to achieve above objects, this invention provides a process for producing light olefins from methanol or dimethyl ether as following:

A process for producing light olefins from methanol or dimethyl ether, comprising the steps of:

1) charging a catalyst pretreating device with a catalyst, and introducing a pretreating gas into the catalyst pretreating device to pretreat the catalyst; wherein the pretreated catalyst contains more than 2% and less than 20% of coke based on the weight of the catalyst, and wherein the prtreating gas is selected from hydrocarbon having 2-6 carbon atoms, an oxygen-containing hydrocarbon compound having 2-6 carbon atoms, and a mixture thereof; and

2) charging a reactor with the pretreated catalyst, introducing methanol or dimethyl ether as a feed into the reactor, and contacting methanol or dimethyl ether with the catalyst in the reactor to react at a reaction temperature of 300-800°C.

In the process for producing light olefins from methanol or dimethyl ether, the pretreating gas in the step 1) further contains auxiliary fluidizing gas.

In the process for producing light olefins from methanol or dimethyl ether, the auxiliary fluidizing gas is selected from nitrogen, steam, argon, hydrogen, methane, and a mixture thereof.

In the process for producing light olefins from methanol or dimethyl ether, the catalyst used is a silicoaluminophosphate molecular sieve catalyst.

In the process for producing light olefins from methanol or dimethyl ether, the pretreating gas used in step 1) is at least one selected from alkane, olefins, alkynes, alcohols, ketones, ethers or alkylene epoxides having 2-6 carbon atoms, or a mixture thereof.

In the process for producing light olefins from methanol or dimethyl ether, the temperature ranges from 300°C to 800°C and the pressure ranges from 0.05 MPa to 1 MPa absolute in the catalyst pretreating device, and the temperature ranges from 350°C to 700°C and the pressure ranges from 0.05 MPa to 1 MPa absolute in the reactor.

In the process for producing light olefins from methanol or dimethyl ether, the temperature ranges from 400°C to 700°C and the pressure ranges from 0.1 MPa to 0.5 MPa absolute in the catalyst pretreating device.

In the process for producing light olefins from methanol or dimethyl ether, the pretreated catalyst contains coke in amount of 2%-7%, based on the weight of the catalyst; and the catalyst after being used in the reactor contains coke in amount of 3%-10%, based on the weight of the catalyst

In the process for producing light olefins from methanol or dimethyl ether, a fresh catalyst, regenerated catalyst, or a mixture thereof is used as a catalyst to be pretreated, which contains coke in amount from 0% to 3%, based on the weight of the catalyst.

The process for producing light olefins from methanol or dimethyl ether further comprises a catalyst regeneration step, and the regenerated catalyst contains coke in amount from 0% to 3%, based on the weight of the catalyst.

The apparatus used for preparing light olefins from methanol or dimethyl ether comprises a catalyst pretreating device. The catalyst pretreating device is preferably constructed as a fluidized bed, and the reactor is constructed as a fluidized bed, moving bed or fixed bed reactors, wherein the reactor is preferably constructed as a fluidized bed reactor; wherein the catalyst pretreating device and the reactor are arranged as separate reactors or integrated in a single reactor.

This invention provides the following advantages and significant effects, compared with the processes in the art. A pretreatment step is added ahead of the reactor so as to pretreat the fresh catalyst or regenerated catalyst, such that the earlier stage of induction period of the catalyst can be overleaped, and the catalyst will be brought into an operation region with high selectivity as soon as it participates in the reaction for producing light olefins from methanol or dimethyl ether. As a result, the process can produce ethylene and propylene in a higher yield.

### Embodiments of the Invention

The catalyst is aluminosilicophosphate (SAPO) molecular sieve catalysts or ZSM series of molecular sieve catalysts. SAPO molecular sieve catalysts are preferable. These catalysts can be obtained as the method presented in CN1088483A. The prtreating gas is at least one selected from alkane, olefins, alkynes, alcohols, ketones, ethers or alkylene epoxides having 2-6 carbons, or a mixture thereof. The pretreated catalyst from step 1) contains coke in amount greater than 2% and less than 15%, preferably greater than 2% and less than 7%. The catalyst after being used in the reactor in step 2) contains coke in amount of 3%-10%.

In the process of this invention, the pretreating gas in step 1) further contains auxiliary fluidizing gas, which is preferably at least one selected from nitrogen, steam, argon, hydrogen, methane, and a mixture thereof.

The aluminosilicophosphate (SAPO) molecular sieve catalyst used in the pretreatment step can be at least one selected from freshly prepared catalyst, regenerated catalyst and a mixture thereof. The catalyst to be treated preferably contains coke in amount from 0% to 3%, preferably 0% to 1%.

As to the temperature in the catalyst pretreating device, a temperature at which the carbon sources can be decomposed into coke but without causing collapse of molecular sieve can be used to deposit the coke onto the surface of molecular sieve. By virtue of the teaching of present invention, a person skilled in the art can determine the temperature through routine tests according to the kinds of carbon sources.

However, in general, when the pretreatment temperature is too low, carbon source is decomposed slowly such that a longer period of time is required for the deposition of a sufficient amount of coke, and when the pretreatment temperature is too high, carbon source is decomposed rapidly resulting in a non-uniform distribution of the deposited coke on the surface of catalyst. In another hand, if the pretreatment temperature is too high, the catalyst can be deactivated permanently because of the collapse of molecular sieve's framework. Therefore, the pretreatment temperature used herein is preferably from 300°C to 800°C, more preferably from 400°C to 700°C.

As to the temperature in the MTO reactor, a temperature at which methanol or dimethyl ether can be reacted on the SAPO molecular sieve catalyst can be used to convert methanol or dimethyl ether to light olefins. By virtue of the teaching of present invention, a person skilled in the art can determine the temperature through routine tests according to the kinds of carbon sources. The reaction temperature in the reactor herein is preferably from 300°C to 800°C, more preferably from 350°C to 700°C.

As to the pressures in the catalyst pretreating device and the reactor, common pressures can be used for practicing the process according to this invention. However, it is difficult to design and operate the reactor if the pressure is too low; and the reactor manufacturing cost and the load of the power system will increase if the pressure is too high. Accordingly, the pressures of the catalyst pretreating device and the reactor used herein are preferably from 0.05 MPa to 1 MPa, more preferably from 0.1 MPa to 0.5 MPa.

There is no specific limit to the catalyst pretreating device, any kind of reactor which can achieve the decomposition of carbon sources and deposition of coke onto molecular sieve, such as a fixed bed reactor, a fluidized bed reactor or a moving bed reactor, are feasible. The catalyst pretreating device used herein is preferably a fluidized bed reactor or a moving bed reactor.

There is no specific limit to the reactor used for preparing light olefins from methanol or dimethyl ether. Any reactor in which methanol or dimethyl ether can be contacted with catalyst to form light olefins, such as a fixed bed reactor, a fluidized bed reactor or a moving bed reactor, can be used to achieve the object of this invention. The reactor used herein is preferably a fluidized bed reactor.

The catalyst pretreating device and the reactor referred to herein can be arranged as separate reactors or integrated as a single reactor. Additionally, a catalyst regenerator can be added to the process to achieve a recycle of the catalyst between the catalyst pretreating device, the reactor and the catalyst regenerator.

Some specific examples are provided hereunder for a better understanding of the technical solutions and technical effects of the present invention.

Example 1:

Catalyst pretreatment: 10 g of fresh SAPO-34 catalyst is charged into a fixed bed reactor having an internal diameter of 30mm. The initial coke content present in the catalyst is 0% based on the weight of the catalyst. The reaction takes place at a temperature of 350°C and at a pressure of 1 MPa absolute. The reactor is first purged by highly pure N₂ for 30 mins at a flow rate of 100 ml/min, and then a pretreating gas having ethylene as its main component is introduced from bottom of the reactor at a mass space velocity of 0.2/h for 20 mins, then the reactor is purged by N₂ for additional 30 mins. The coke content present in the pretreated catalyst is shown in Table 1.

MTO reaction: the pretreated catalyst is charged into a quartz tube fluidized-bed reactor having an internal diameter of 20mm. Reaction temperature is maintained at 450°C and reaction pressure is maintained at 0.15 MPa absolute. Methanol used as feed stock is introduced into the reactor after being vaporized by a pre-heater. The mass space velocity of methanol is 3/h relative to the catalyst. The products from the reactor outlet are condensed by a condenser. The condensed reaction gas is recovered by a gas collecting bottle. The liquid components in the products are analyzed on-line for concentration of methanol. The reaction is continued until the mass concentration of methanol in the liquid effluent is 4%. The gas in the gas collecting bottle is analyzed by gas chromatography for the content of hydrocarbons. The selectivity to ethylene and propylene in gas products, as well as the coke content present in the used catalyst are shown in Table 1.

Example 2:

Catalyst pretreatment: 10 g of regenerated SAPO-34 catalyst is charged into a quartz tube fluidized-bed reactor having an internal diameter of 20 mm. The initial coke content present in the catalyst is 0.1% based on the weight of the catalyst. The reaction takes place at a temperature of 450°C and at a pressure of 0.15 MPa absolute. The reactor is first purged by highly pure N2 for 30 mins at a flow rate of 300ml/min, and then a pretreating gas having propylene as its main component is introduced from bottom of the reactor at a mass space velocity of 1.5/h for 8 mins, then the reactor is purged by N2 for additional 30 mins. The coke content present in the pretreated catalyst is shown in Table 1.

The MTO reaction condition are the same as those in example 1. The selectivity to ethylene and propylene in gas products, as well as the coke content present in the used catalyst are shown in Table 1.

Example 3:

Catalyst pretreatment: 10 g of regenerated SAPO-34 catalyst is charged into a quartz tube fluidized-bed reactor having an internal diameter of 20 mm. The initial coke content present in the catalyst is 0.1% based on the weight of the catalyst. The reaction takes place at a temperature of 500°C and at a pressure of 0.15 MPa absolute. The reactor is first purged by highly pure N2 for 30 mins at a flow rate of 300ml/min, and then a pretreating gas having 1- butylene as its main component is introduced from bottom of the reactor at a mass space velocity of 2/h for 5 mins, then the reactor is purged by highly pure N2 for additional 30 mins. The coke content present in the pretreated catalyst is shown in Table 1.

The MTO reaction conditions are the same as those in example 1. The selectivity to ethylene and propylene in gas products, as well as the coke content present in the used catalyst are shown in Table 1.

Example 4:

Catalyst pretreatment: 10 g of regenerated SAPO-34 catalyst is charged into a quartz tube fluidized-bed reactor having an internal diameter of 20 mm. The initial coke content present in the catalyst is 0.1% based on the weight of the catalyst. The reaction takes place at a temperature of 550°C and at a pressure of 0.15 MPa absolute. The reactor is first purged by highly pure N2 for 30 mins at a flow rate of 300ml/min, and then a pretreating gas consisting of a mixture of 4.1 vol. % butane, 30.3 vol.% butylene, 3.2 vol.% pentane, 15.7 vol.% pentene, 0.8 vol.% hexane, 3.5 vol.% hexene, 23.1 vol.% methane, and 19.3 vol.% hygrogen is introduced from bottom of the reactor at a mass space velocity of 1.5/h for 8 mins, then the reactor is purged by highly pure N2 for additional 30 mins. The coke content present in the pretreated catalyst is shown in Table 1.

The MTO reaction conditions are the same as those in example 1. The selectivity to ethylene and propylene in gas products, as well as the coke content present in the used catalyst are shown in Table 1.

Example 5:

Catalyst pretreatment: 10 g of regenerated SAPO-34 catalyst is charged into a quartz tube fluidized-bed reactor having an internal diameter of 20 mm. The initial coke content present in the catalyst is 0.1% based on the weight of the catalyst. The reaction takes place at a temperature of 600°C and at a pressure of 0.15 MPa absolute. The reactor is first purged by highly pure N2 for 30 mins at a flow rate of 300ml/min, and then a pretreating gas consisting of a mixture of 69.5 vol.% ethane, 12 vol.% ethylene, 0.5 vol.% ethylene oxide, 17.7 vol.% propane, and 0.3 vol.% acetone is introduced from bottom of the reactor at a mass space velocity of 1.5/h for 12 mins, then the reactor is purged by highly pure N2 for additional 30 mins. The coke content present in the pretreated catalyst is shown in Table 1.

The MTO reaction conditions are the same as those in example 1. The selectivity to ethylene and propylene in gas products, as well as the coke content present in the used catalyst are shown in Table 1.

Example 6:

Catalyst pretreatment: 10 g of regenerated SAPO-34 catalyst is charged into a quartz tube fluidized-bed reactor having an internal diameter of 20 mm. The initial coke content present in the catalyst is 0.1% based on the weight of the catalyst. The reaction takes place at a temperature of 650°C and at a pressure of 0.15 MPa absolute. The reactor is first purged by highly pure N2 for 30 mins at a flow rate of 300ml/min, and then a pretreating gas consisting of methanol is introduced from bottom of the reactor at a mass space velocity of 4/h for 9 mins, then the reactor is purged by highly pure N2 for additional 30 mins. The coke content present in the pretreated catalyst is shown in Table 1.

The MTO reaction conditions are the same as those in example 1. The selectivity to ethylene and propylene in gas products, as well as the coke content present in the used catalyst are shown in Table 1.

Example 7:

Catalyst pretreatment: 10 g of regenerated SAPO-34 catalyst is charged into a quartz tube fluidized-bed reactor having an internal diameter of 20 mm. The initial coke content present in the catalyst is 0.1% based on the weight of the catalyst. The reaction takes place at a temperature of 700°C and at a pressure of 0.15 MPa absolute. The reactor is first purged by highly pure N2 for 30 mins at a flow rate of 300ml/min, and then a pretreating gas consisting of dimethyl ether is introduced from bottom of the reactor at a mass space velocity of 2.5/h for 12 mins, then the reactor is purged by highly pure N2 for additional 30 mins. The coke content present in the pretreated catalyst is shown in Table 1.

The MTO reaction conditions are the same as those in example 1. The selectivity to ethylene and propylene in gas products, as well as the coke content present in the used catalyst are shown in Table 1.

Example 8:

Catalyst pretreatment: 10 g of regenerated SAPO-34 catalyst is charged into a quartz tube fluidized-bed reactor having an internal diameter of 20 mm. The initial coke content present in the catalyst is 1% based on the weight of the catalyst. The reaction takes place at a temperature of 800°C and at a pressure of 0.1 MPa absolute. The reactor is first purged by highly pure N2 for 30 mins at a flow rate of 300ml/min, and then a pretreating gas consisting of a mixture of 25 vol.% ethanol, 25 vol.% propanol, and 50 vol.% steam is introduced from bottom of the reactor at a mass space velocity of 1.5/h for 15 mins, then the reactor is purged by highly pure N2 for additional 30 mins. The coke content present in the pretreated catalyst is shown in Table 1.

The MTO reaction conditions are the same as those in example 1. The selectivity to ethylene and propylene in gas products, as well as the coke content present in the used catalyst are shown in Table 1.

Example 9:

Catalyst pretreatment: 100 g of fresh SAPO-34 catalyst is charged into a fluidized-bed reactor having an internal diameter of 50mm. The initial coke content present in the catalyst is 0% based on the weight of the catalyst. The pretreatment is performed at a temperature of 450°C and at a pressure of 0.1 MPa absolute. The reactor is first purged by highly pure N2 for 30 mins at a flow rate of 60L/h, and then a pretreating gas consisting of propylene is introduced from bottom of the reactor at a mass space velocity of 1/h for 10 mins, then the reactor is purged by highly pure N2 for additional 30 mins. The coke content present in the pretreated catalyst is 3.4% based on the weight of the catalyst.

MTO reaction: the pretreated catalyst is charged into a stainless steel fluidized-bed reactor having an internal diameter of 50mm. Reaction temperature is maintained at 500°C and reaction pressure is maintained at 0.1 MPa absolute. Methanol used as feed stock is introduced into the reactor after being vaporized by a pre-heater. The mass space velocity of methanol is 3/h relative to the catalyst. The products from the reactor outlet are condensed by a condenser. The condensed reaction gas is recovered by a gas collecting bottle. The liquid components in the products are analyzed on-line for concentration of methanol. The reaction is continued until the mass concentration of methanol in the liquid effluent is 4%. The gas in the gas collecting bottle is analyzed by gas chromatography for the content of hydrocarbons. The selectivity to ethylene and propylene (collectively referred to as "the two olefins") in gas products is 82.1%, and the coke content present in the used catalyst is 7.6% based on the weight of the catalyst.

Example 10:

Catalyst pretreatment: 100 g of regenerated SAPO-34 catalyst is charged into a moving bed reactor having an internal diameter of 50mm. The initial coke content present in the catalyst is 0.05% based on the weight of the catalyst. The pretreatment is performed at a temperature of 500°C and at a pressure of 0.15 MPa absolute. The reactor is first purged by highly pure N2 for 30 mins at a flow rate of 10L/h, and then a pretreating gas consisting of dimethyl ether is introduced from bottom of the reactor at a mass space velocity of 0.2/h for 40 mins, then the reactor is purged by highly pure N2 for additional 30 mins. The coke content present in the pretreated catalyst is 7% based on the weight of the catalyst.

MTO reaction: the pretreated catalyst is charged into a stainless steel fluidized-bed reactor having an internal diameter of 50mm. Reaction temperature is maintained at 430°C and reaction pressure is maintained at 0.15 MPa absolute. Methanol used as feed stock is introduced into the reactor after being vaporized by a pre-heater. The mass space velocity of methanol is 3/h relative to the catalyst. The products from the reactor outlet are condensed by a condenser. The condensed reaction gas is recovered by a gas collecting bottle. The liquid components in the products are analyzed on-line for concentration of methanol. The reaction is continued until the mass concentration of methanol in the liquid effluent is 4%. The gas in the gas collecting bottle is analyzed by gas chromatography for the content of hydrocarbons. The selectivity to ethylene and propylene (collectively referred to as "the two olefins") in gas products is 83%, and the coke content present in the used catalyst is 8% based on the weight of the catalyst.

Example 11:

Catalyst pretreatment: 100 g of regenerated SAPO-34 catalyst is charged into a fluidized bed reactor having an internal diameter of 50mm. The initial coke content present in the catalyst is from 0.1% to 0.3% based on the weight of the catalyst. The pretreatment is performed at a temperature of 450°C and at a pressure of 0.15 MPa absolute. The reactor is first purged by highly pure N2 for 30 mins at a flow rate of 60L/h, and then a pretreating gas consisting of 50 vol.% 1-butylene and 50 vol.% steam is introduced from bottom of the reactor at a mass space velocity of 2/h for 10 mins, then the reactor is purged by highly pure N2 for additional 30 mins.. The coke content present in the pretreated catalyst is from 3.1% to 3.7% based on the weight of the catalyst.

MTO reaction: the pretreated catalyst is charged into a stainless steel fluidized-bed reactor having an internal diameter of 50mm. Reaction temperature is maintained at 550°C and reaction pressure is maintained at 0.15 MPa absolute. Methanol used as feed stock is introduced into the reactor after being vaporized by a pre-heater. The mass space velocity of methanol is 3/h relative to the catalyst. The products from the reactor outlet are condensed by a condenser. The condensed reaction gas is recovered by a gas collecting bottle. The liquid components in the products are analyzed on-line for concentration of methanol. The reaction is continued until the mass concentration of methanol in liquid effluent is 4%. The gas in the gas collecting bottle is analyzed by gas chromatography for the content of hydrocarbons. The selectivity to ethylene and propylene (collectively referred to as "the two olefins") in gas products is from 81.7% to 82.8%, and the coke content present in the used catalyst is from 7.2% to 7.6% based on the weight of the catalyst.

Catalyst regeneration: 100 g of used catalyst is charged into a stainless steel fluidized bed reactor having an internal diameter of 50mm. Regeneration temperature is maintained at 700°C, regeneration pressure is maintained at 0.15 MPa absolute, and air used as regenerating gas is introduced at a flow rate of 60L/h for 1h. The coke content present in the regenerated catalyst is from 0.1% to 0.3% on an average based on the weight of the catalyst.

The regenerated catalyst is charged into the catalyst pretreating device for use in recycle

Comparative example 1:

10 g of fresh catalyst is charged into a quartz tube fluidized-bed reactor having an internal diameter of 20mm. The initial coke content present in the catalyst is from 0% based on the weight of the catalyst. Reaction temperature is at 450°C and reaction pressure is 0.15 MPa absolute. The reactor is first purged by highly pure N2 for 30 mins at a flow rate of 300ml/min. Methanol used as feed stock is introduced into the reactor after being vaporized by a pre-heater. The mass space velocity of methanol is 3/h relative to the catalyst. The products from the reactor outlet are condensed by a condenser. The condensed reaction gas is recovered by a gas collecting bottle. The liquid components in the products are analyzed on-line for concentration of methanol. The reaction is continued until the mass concentration of methanol in the liquid effluent is 4%. The gas in the gas collecting bottle is analyzed by gas chromatography for the content of hydrocarbons. The selectivity to ethylene and propylene in gas products is 78.2%, and the coke content present in the used catalyst is 7.4% based on the weight of the catalyst.

Comparative example 2:

MTO reaction: the regenerated catalyst is charged into a stainless steel fluidized-bed reactor having an internal diameter of 50mm. Reaction temperature is maintained at 500°C and reaction pressure is maintained at 0.15 MPa absolute. Methanol used as feed stock is introduced into the reactor after being vaporized by a pre-heater. The mass space velocity of methanol is 3/h relative to the catalyst. The products from the reactor outlet are condensed by a condenser. The condensed reaction gas is recovered by a gas collecting bottle. The liquid components in the products are analyzed on-line for concentration of methanol. The reaction is continued until the mass concentration of methanol in the liquid effluent is 4%. The gas in the gas collecting bottle is analyzed by gas chromatography for the content of hydrocarbons. The selectivity to ethylene and propylene (collectively referred to as "the two olefins") in gas products is from 78.1% to 78.7%, and the coke content present in the used catalyst is from 7.3% to 7.6% based on the weight of the catalyst.

Catalyst regeneration: the used catalyst is charged into a stainless steel fluidized bed reactor having an internal diameter of 50mm. Regeneration temperature is maintained at 600°C and regeneration pressure is maintained at 0.15 MPa absolute, and air used as regenerating gas is introduced at a flow rate of 60L/h for 1h. The coke content present in the regenerated catalyst is from 0.1% to 0.3% on an average based on the weight of the catalyst.

It can be seen from above examples and Comparative examples that the process for producing light olefins from methanol or dimethyl ether according to this invention can be performed at a higher selectivity to the ethylene and propylene.

Table 1

| No. | The coke content present in the pretreated catalyst (wt%) | Selectivity to the ethylene and propylene in the products from the reactor outlet (%) | The coke content present in the used catalyst after reaction (wt%) |
|---|---|---|---|
| example 1 | 2.1 | 80.1 | 7.4 |
| example 2 | 2.8 | 81.6 | 7.5 |
| example 3 | 3.6 | 82.3 | 7.3 |
| example 4 | 4.5 | 83.8 | 7.5 |
| example 5 | 5.3 | 83.5 | 7.4 |
| example 6 | 5.8 | 83.3 | 7.6 |
| example 7 | 6.2 | 83.2 | 7.5 |
| example 8 | 6.3 | 84.1 | 7.8 |

## Claims

1. A process for producing light olefins from methanol or dimethyl ether, comprising the steps of:
1) charging a catalyst pretreating device with a catalyst, and introducing a pretreating gas into the catalyst pretreating device to pretreat the catalyst; wherein the pretreated catalyst contains more than 2% and less than 15% of coke based on the weight of the catalyst, and wherein the prtreating gas is selected from hydrocarbon having 2-6 carbon atoms, an oxygen-containing hydrocarbon compound having 2-6 carbon atoms, and a mixture thereof; and
2) charging a reactor with the pretreated catalyst, introducing methanol or dimethyl ether as a feed into the reactor, and contacting methanol or dimethyl ether with the catalyst in the reactor to react at a reaction temperature of 300-800°C.

2. A process for producing light olefins from methanol or dimethyl ether according to claim 1, **characterized in that** the pretreating gas in the step 1) further contains auxiliary fluidizing gas.

3. A process for producing light olefins from methanol or dimethyl ether according to claim 2, **characterized in that** the auxiliary fluidizing gas is selected from nitrogen, steam, argon, hydrogen, methane, and a mixture thereof.

4. A process for producing light olefins from methanol or dimethyl ether according to claim 1, **characterized in that** the catalyst used is a silicoaluminophosphate molecular sieve catalyst.

5. A process for producing light olefins from methanol or dimethyl ether according to claim 1, **characterized in that** the pretreating gas used in step 1) is at least one selected from alkane, olefins, alkynes, alcohols, ketones, ethers or alkylene epoxides having 2-6 carbon atoms, or a mixture thereof.

6. A process for producing light olefins from methanol or dimethyl ether according to any one of claims 1 to 5, **characterized in that** the temperature ranges from 300°C to 800°C and the pressure ranges from 0.05 MPa to 1 MPa absolute in the catalyst pretreating device, and the temperature ranges from 350°C to 700°C and the pressure ranges from 0.05 MPa to 1 MPa absolute in the reactor.

7. A process for producing light olefins from methanol or dimethyl ether according to claim 6, **characterized in that** the temperature ranges from 400°C to 700°C and the pressure ranges from 0.1 MPa to 0.5 MPa absolute in the catalyst pretreating device.

8. A process for producing light olefins from methanol or dimethyl ether according to any one of claims 1 to 5, **characterized in that** the pretreated catalyst contains coke in amount of 2%-7%, based on the weight of the catalyst; and the catalyst after being used in the reactor contains coke in amount of 3%-10%, based on the weight of the catalyst.

9. A process for producing light olefins from methanol or dimethyl ether according to any one of claims 1 to 5, **characterized in that** a fresh catalyst, regenerated catalyst, or a mixture thereof is used as a catalyst to be pretreated, which contains coke in amount from 0% to 3%, based on the weight of the catalyst.

10. A process for producing light olefins from methanol or dimethyl ether according to any one of claims 1 to 5, **characterized in that** the process further comprises a catalyst regeneration step, and the regenerated catalyst contains coke in amount from 0% to 3%, based on the weight of the catalyst.

11. A process for producing light olefins from methanol or dimethyl ether according to any one of claims 1 to 5, **characterized in that** the catalyst pretreating device is constructed as a fluidized bed and the reactor is constructed as a fluidized bed, moving bed or fixed bed reactors, wherein the reactor is preferably constructed as a fluidized bed reactor; wherein the catalyst pretreating device and the reactor are arranged as separate reactors or integrated as a single reactor.
